# EUROPÄISCHE PATENTSCHRIFT

(11) **EP 1 909 207 B1**
(45) Veröffentlichungstag und Bekanntmachung des Hinweises auf die Patenterteilung: **04.03.2015**
(21) Anmeldenummer: 07017673.0
(22) Anmeldetag: 10.09.2007
(51) Int. Cl.: G01N 33/543

(54) **Messbereichserweiterung chromatografischer Schnellteste**
Measuring range extension of chromatographic quick tests
Agrandissement de l'étendue de mesure d'essais chromatographiques rapides

(30) Priorität: 11.09.2006 EP 06019008
(43) Veröffentlichungstag der Anmeldung: 09.04.2008
(73) Patentinhaber: F.Hoffmann-La Roche AG, 4070 Basel (CH); Roche Diagnostics GmbH, 68305 Mannheim (DE)
(72) Erfinder: Spinke, Jürgen, 64653 Lorsch (DE); Thiele, Marcel, 68309 Mannheim (DE); Schäffler, Jürgen, 69469 Weinheim (DE); Nufer, Andreas, 67098 Bad Dürkheim (DE)
(74) Vertreter: Weiss, Wolfgang

(56) Entgegenhaltungen:
- WO-A-96/24062
- WO-A2-00/45176
- US-A1- 2006 089 810
- SHIN IN SOO ET AL: "An improved, reliable and practical kinetic assay for the detection of prekallikrein activator in blood products." ARCHIVES OF PHARMACAL RESEARCH (SEOUL), Bd. 25, Nr. 4, August 2002 (2002-08), Seiten 505-510, XP009078638 ISSN: 0253-6269
- LAGERSTEDT SUSAN A ET AL: "Measurement of plasma free metanephrine and normetanephrine by liquid chromatography-tandem mass spectrometry for diagnosis of pheochromocytoma." CLINICAL CHEMISTRY MAR 2004, Bd. 50, Nr. 3, März 2004 (2004-03), Seiten 603-611, XP002419018 ISSN: 0009-9147

## Beschreibung

Die vorliegende Erfindung betrifft Verfahren und Vorrichtungen zur quantitativen Bestimmung eines Analyten in einer Probe.

Ein weitverbreitetes Analyseverfahren zur schnellen Bestimmung von Analyten wie beispielsweise Drogen, Schwangerschaftshormonen, Infektionskrankheiten oder CARDIAC-Markern greift auf die Verwendung immunologischer Teststreifen zurück. Dabei finden sowohl qualitative Tests, welche rein visuell ausgelesen werden (z.B. CARDIAC D-Dimer, Trop T sensitive, etc.) als auch quantitative Tests, welche mittels eines Auslesegeräts ausgewertet werden (z.B. Elecsys® proBNP, Roche CARDIAC proBNP, etc.), breite Anwendung.

Derartige quantitative immunologische Teststreifen zeichnen sich insbesondere durch ihre leichte Handhabbarkeit aus. Die Teststreifen basieren im Allgemeinen darauf, dass im Teststreifen ein Reagenz enthalten ist, welches durch Reaktion mit dem Analyten in der Probe zu einem nachweisbaren Signal führt. Das nachweisbare Signal wird dabei im Allgemeinen über Remissionsmessung nach einem festgelegten Zeitraum bestimmt. Der Zeitraum zwischen Inkontaktbringen von Analyt und Reagenz und der Signalmessung wird dabei möglichst lang gewählt. Hierdurch wird eine lange Reaktionszeit zwischen Reagenz und Analyt und somit eine möglichst hohe Empfindlichkeit solcher Teststreifen gewährleistet. Aus reaktionskinetischen Gründen ist jedoch nach einer derartigen langen Reaktionszeit eine quantitative Bestimmung von Analyten, die in einer hohen Konzentration in einer Probe vorliegen nicht mehr möglich.

Gegenüber herkömmlichen Laboranalysesystemen, wie z.B. Elecsys (Roche Diagnostics), IM (Abbott), Dimension (Dade Behring), weisen derartige Teststreifen deshalb noch deutliche Schwächen hinsichtlich ihrer Leistungsfähigkeit auf. Besonders die Messgenauigkeit und der dynamische Messbereich sind bei Teststreifen beispielsweise im Vergleich zu Reaktionen in Lösung deutlich verschlechtert. Dies limitiert ihre Anwendung bei der Bestimmung von Analyten, welche sowohl eine hohe Sensitivität als auch einen möglichst großen Messbereich erfordern. Insbesondere bei der Notfallversorung von Patienten wäre es für den behandelnden Arzt sehr hilfreich, wenn Test oder ein Verfahren bereitgestellt werden könnte, durch welches aufgrund seiner hohen Sensitivität einerseits bestimmte Erkrankungen sicher ausgeschlossen werden könnten, andererseits aber auch ein großer Messbereich bereitgestellt werden würde. Ein großer Messbereich für einen Analyten ist insbesondere bei der Risikostratifizierung und beim Therapie-Monitoring wünschenswert. Eine Messbereicherweitung von Tests wäre insbesondere für solche pathologische Zustände wünschenswert, bei denen die Konzentration eines für den Zustand charakteristischen Analyten oder Markers mit der Schwere des Erkrankungszustands korreliert. In solchen Fällen kann eine erhöhte Markerkonzentration (z.B. NT-proBNP) auf eine erhöhte Gefahrensituation für einen Patienten hinweisen.

Eine der Erfindung zugrunde liegende Aufgabe bestand deshalb darin, ein Verfahren zur Erweiterung des quantitativen Messbereichs eines Analyten in einer Probe zur Verfügung zu stellen.

Diese Aufgabe wird gelöst durch die quantitative Bestimmung eines Analyten in einer Probe umfassend
(a) Bereitstellen einer Analyt-spezifischen Substanz, die in der Lage ist, bei Kontakt mit dem Analyten eine Reaktion einzugehen, welche ein nachweisbares Signal erzeugt, wobei zum Bereitstellen der Analyt-spezifischen Substanz ein Teststreifen verwendet wird,
(b) Vorgeben von wenigstens zwei Kalibrierungsgraphen, die durch Reaktion der jeweils gleichen Analyt-spezifischen Substanz mit unterschiedlichen Mengen eines jeweils gleichen Test-Analyten für jeweils eine vorbestimmte Reaktionszeit erstellt worden sind,
(c) Inkontaktbringen der Analyt-spezifischen Substanz mit einer Probe, die den nachzuweisenden Analyten umfasst,
(d) Messen des Signals zu einer ersten vorbestimmten Reaktionszeit, für die ein erster Kalibrierungsgraph gemäß (b) vorgegeben ist,
(e) Überprüfen, ob das gemäß (d) gemessene Signal eine quantitative Bestimmung des Analyten mit einer gewünschten Genauigkeit ermöglicht, wobei die gewünschte Genauigkeit für die zu bestimmende Analytmenge erreicht ist, wenn der betrachtete Kalibierungsgraph in Bezug auf die entsprechende Menge Test-Analyt die größte Steigung aller vorgegebenen Kalibierungskurven aufweist,
(f)
   (i) quantitatives Bestimmen des Analyten auf Basis des gemäß (d) gemessenen Signals, wenn die gewünschte Genauigkeit erreicht ist, oder
   (ii) Messen des Signals zu einer zweiten vorbestimmten Reaktionszeit, für die ein zweiter Kalibrierungsgraph gemäß (b) vorgegeben ist,
(g) Überprüfen, ob das gemäß (f)(ii) gemessene Signal eine quantitative Bestimmung des Analyten mit einer gewünschten Genauigkeit ermöglicht, wobei die gewünschte Genauigkeit für die zu bestimmende Analytmenge erreicht ist, wenn der betrachtete Kalibierungsgraph in Bezug auf die entsprechende Menge Test-Analyt die größte Steigung aller vorgegebenen Kalibierungskurven aufweist, und
(h)
   (i) quantitatives Bestimmen des Analyten auf Basis des gemäß (f)(ii) gemessenen Signals, wenn die gewünschte Genauigkeit erreicht ist, oder
   (ii) Fortsetzen der Bestimmung bei zumindest einer weiteren vorbestimmten Reaktionszeit entsprechend (f) (ii), (g), (h)(i),
   d.h. gegebenenfalls
(i) Messen des Signals zu einer dritten vorbestimmten Reaktionszeit, für die ein dritter Kalibrierungsgraph gemäß (b) vorgegeben ist,
(j) Überprüfen, ob das gemäß (i) gemessene Signal eine quantitative Bestimmung des Analyten mit einer gewünschten Genauigkeit ermöglicht, wobei die gewünschte Genauigkeit für die zu bestimmende Analytmenge erreicht ist, wenn der betrachtete Kalibierungsgraph in Bezug auf die entsprechende Menge Test-Analyt die größte Steigung aller vorgegebenen Kalibierungskurven aufweist, und
(k)
   (i) quantitatives Bestimmen des Analyten auf Basis des gemäß (i) gemessenen Signals, wenn die gewünschte Genauigkeit erreicht ist, oder
   (ii) Fortsetzen der Bestimmung bei zumindest einer weiteren vorbestimmten Reaktionszeit.

Die Schritte (f)(ii), (g) und (h)(i) des Verfahrens können beliebig oft wiederholt werden. In einer bevorzugten Ausführungsform werden diese Schritt zwei oder dreimal wiederholt, d.h. es werden entsprechend zwei oder drei Kalibrierungsgraphen für zwei oder drei vorbestimmte Reaktionszeiten erstellt bzw. vorgegeben.

Ein weiterer Aspekt der vorliegenden Erfindung betrifft ein Verfahren zur quantitativen Bestimmung eines Analyten in einer Probe umfassend
(a) Bereitstellen einer Analyt-spezifischen Substanz, die in der Lage ist, bei Kontakt mit dem Analyten eine Reaktion einzugehen, welche ein nachweisbares Signal erzeugt, wobei zum Bereitstellen der Analyt-spezifischen Substanz ein Teststreifen verwendet wird,
(b) Vorgeben von wenigstens zwei Kalibrierungsgraphen, die durch Reaktion der jeweils gleichen Analyt-spezifischen Substanz mit unterschiedlichen Mengen eines jeweils gleichen Test-Analyten für jeweils eine vorbestimmte Reaktionszeit erstellt worden sind,
(c) Inkontaktbringen der Analyt-spezifischen Substanz mit einer Probe, die den nachzuweisenden Analyten umfasst,
(d) Messen eines ersten Signals zu einer ersten vorbestimmten Reaktionszeit, für die ein erster Kalibrierungsgraph gemäß (b) vorgegeben ist,
(e) Messen eines zweiten Signals zu einer zweiten vorbestimmten Reaktionszeit, für die ein zweiter Kalibrierungsgraph gemäß (b) vorgegeben ist,
(f) gegebenenfalls Messen eines weiteren Signals,
(g) Überprüfen, welches der gemäß (d), (e) oder (f) gemessenen Signale eine ausreichende Genauigkeit bei der quantitativen Bestimmung des Analyten ermöglicht, wobei die gewünschte Genauigkeit für die zu bestimmende Analytmenge erreicht ist, wenn der betrachtete Kalibierungsgraph in Bezug auf die entsprechende Menge Test-Analyt die größte Steigung aller vorgegebenen Kalibierungskurven aufweist, und
(h) quantitatives Bestimmen des Analyten auf Basis des Signals, welches eine ausreichende Genauigkeit ermöglicht.

Um zu überprüfen, ob das erste gemessene oder zweite gemessene Signal eine quantitative Bestimmung des Analyten mit einer größeren Genauigkeit ermöglicht, wird in einer bevorzugten Ausführungsform basierend auf den wenigstens zwei vorgegebenen Kalibrierungsgraphen ein empirischer Konzentrationsgrenzwert festgelegt. Analyt-Konzentrationen, welche diesen Grenzwert übersteigen, werden nach der kürzeren der beiden Reaktionszeiten ausgewertet, während Analyt-Konzentrationen, die diesen Grenzwert unterschreiten, nach der längeren der beiden Reaktionszeiten bestimmt werden. Wird festgestellt, dass die Analyt-Konzentration den Grenzwert nach der kurzen Reaktionszeit übersteigt, also eine hohe Analyt-Konzentration bestimmt wird, kann das Verfahren zu diesem Zeitpunkt beendet werden.

Überraschenderweise wurde festgestellt, dass es durch die erfindungsgemäßen Verfahren im Vergleich zu aus dem Stand der Technik bekannten Verfahren möglich ist, die obere Messbereichsgrenze um mehr als das Dreifache zu erhöhen. Die erfindungsgemäßen Verfahren verbessern somit die diagnostische Kompetenz eines behandelnden Arztes.

Durch den erfindungsgemäß erweiterten Messbereich eines Tests kann gegebenenfalls auf zusätzliche, oft aufwändige Tests (z.B. invasive Diagnoseverfahren etc.) verzichtet werden.

Wie hierin später ausführlich beschrieben, ermöglichen die erfindungsgemäße Verfahren insbesondere bei hohen Analytkonzentrationen in einer Probe eine schnellere Konzentrationsbestimmung als Verfahren oder Tests wie sie im Stand der Technik beschrieben werden. Da beispielsweise die Blutspiegel von NT-proBNP mit dem Ausmaß der kardialen Dysfunktion korrelieren, erlauben die erfindungsgemäßen Verfahren eine schnellere Beurteilung des kardiospezifischen Zustands eines Patienten in Notfallsituationen. Ein sich daraus ergebender Vorteil ist, dass bei Vorliegen akuter kardialer Ereignisse wie beispielsweise einem akuten Myokartinfarkt Patienten zu einem früheren Zeitpunkt identifiziert und adäquat behandelt werden können als dies bei gegenwärtigen diagnostischen Vorgehen der Fall ist. Durch die erfindungsgemäßen Verfahren und die schnellere Diagnosestellung insbesondere im Fall eines akuten kardialen Ereignisses können durch den behandelnden Arzt schneller entsprechende Gegenmaßnahmen eingeleitet werden und andere kardiale Komplikationen sowie die Häufigkeit von Todesfällen reduziert werden.

In einer bevorzugten Ausführungsform der vorliegenden Erfindung wird eine flüssige, vorzugsweise aus Körperflüssigkeit stammende Probe verwendet. Insbesondere bevorzugt wird eine Blut-, Plasma-, Serum-, Speichel- oder Urinprobe verwendet.

Der quantitativ zu bestimmende Analyt ist vorzugsweise ausgewählt aus Nukleinsäuren, Lipiden, Kohlenhydraten Proteinen, und insbesondere aus DNA, RNA, Antikörpern, Antigenen, Stoffwechselprodukten, Hormonen Viren, Mikroorganismen, Zellen, kardiospezifischen Markern, neurohormonalen Markern, ischämischen Markern und muskelspezifischen Markern.

Bevorzugte Beispiele für Lipide umfassen Cholesterin, HDL-Cholesterin und Triglyceride. Ein besonders bevorzugter Kohlenhydrat-Analyt ist Glucose. Beispiele für zu bestimmende Enzyme umfassen alkalische Phosphatase und Amylase. Harnsäure, Bilirubin und Urobilinogen sind bevorzugte Beispiele für Stoffwechselprodukte.

Beispiele für neurohormonale Marker umfassen atriales (A-Typ) natriuretisches Peptid (ANP), B-Typ natriuretisches Peptid (BNP) oder N-terminalen Fragmente der jeweiligen proPeptide NT-ProANP und NT-ProBNP.

Beispiele für ischämische Marker umfassen Ischämisch Modifiziertes Albumin (IMA), Fettsäure-Bindeprotein (fatty acid binding protein), Freie Fettsäure (free fatty acid), Schwangerschaft-assoziiertes Plasma-Protein A (Pregnancy Associated Plasma Protein A), Glycogenphosphorylase Isoenzym BB sowie Sphingosin-1-Phosphat.

Myoglobin und Creatin-Kinase-MB (CK-MB) sind bevorzugte Beispiele muskelspezifischer Marker.

CD 40 ist ein bevorzugtes Beispiel eines Marker zur Plättchenaktivierung.

Bevorzugte kardiospezifische ischämisch-nekrotische Marker sind Troponin T oder Troponin I.

In einer besonders bevorzugten Ausführungsform wird mindestens ein CARDIAC-Marker bzw. kardiospezifischer Marker bestimmt, welcher vorzugsweise wiederum ausgewählt ist aus Troponin T, Myoglobin, D-Dimer und NT-proBNP.

Die Analyt-spezifische Substanz ist vorzugsweise ausgewählt aus mit dem Analyt bindefähigen Rezeptoren, Antikörpern, Antigenen, Lectin, Nukleinsäuren und Nukleinsäureanaloga. Vorzugsweise ist die Analyt-spezifische Substanz ferner mit einem Nachweisreagenz oder einem Enzym gekoppelt, welche bei Bindung an den Analyten ein nachweisbares Signal erzeugen. In einer bevorzugten Ausführungsform führt die Bindung des Analyten an die Analyt-spezifische Substanz durch Reaktion direkt zu einem nachweisbaren Signal. In einer weiteren Ausführungsform kann nach der Bindung des Analyten an die Analyt-spezifische Substanz ein Substrat zugegeben werden, welches unter Abgabe eines nachweisbaren Signals entweder vom Analyten oder von der Analyt-spezifischen Substanz umgesetzt wird. Bevorzugte Nachweissysteme sind z.B. kolloidale Metallpartikel, insbesondere Gold, fluoreszierende Nanopartikel, z.B. Latex, up-converting Phosphas, Quantum Dots, oder super-paramagnetische Partikel.

Der Nachweis der erfindungsgemäß bevorzugt bestimmten Analyten BNP oder NT-proBNP ist beispielsweise bei Richards et al., (Circulation 97(1998) 1921-1929), Struthers (Eur. Heart J. 20 (1999), 1374-1375), Hunt et al., Clin. Endocrinol. 47 (1997, 287-296), Talwar et al. (Eur. Heart. J. 20 (1999), 1736-1744), Darba et al. (Am. J. Cardiol. 78 (1996), 286-287) sowie in EP-0 648 228 und WO 00/45176 beschrieben.

In einer bevorzugten Ausführungsform ist die Reaktion zwischen Analyt und Analyt-spezifischer Substanz eine Immunreaktion.

Unter "Kalibrierungsgraph" im Sinne der vorliegenden Erfindung wird eine Funktion verstanden, welche sich durch Zuordnen definierter Test-Analytmengen zu definierten, das nachweisbare Signal beschreibenden Parametern ergibt. Eine definierte Test-Analytenmenge wird dabei einem ein definiertes Signal beschreibenden Parameter zugeordnet. Zur Erstellung des Kalibrierungsgraphen können auch Durchschnittswerte, welche sich aus mehreren, vorzugsweise unabhängigen Messungen ergeben, verwendet werden.

Das nachweisbare Signal beschreibende Parameter sind vorzugsweise Parameter, welche eine Adsorption oder Emission von Licht beliebiger Wellenlänge als Folge der Reaktion des Analyten mit der Analyt-spezifischen Substanz beschreiben. Bevorzugte Beispiele das Signal beschreibender Parameter sind Remissions-, Emissions- und Adsorptionswerte. Weiterhin können z.B. Mangnetpartikel verwendet werden, so dass als das Signal beschreibende Parameter auch Magnetfelder (Magnetfeldzustände) in Frage kommen.

Die das nachweisbare Signal beschreibenden Parameter werden vorzugsweise durch Reaktion der jeweils gleichen Analyt-spezifischen Substanz mit unterschiedlichen Mengen eines jeweils gleichen Test-Analyten für jeweils eine vorbestimmte Reaktionszeit gemessen. Hierzu wird die jeweilige Menge des jeweils gleichen Test-Analyten mit der jeweils gleichen Analyt-spezifischen Substanz zur Reaktion gebracht und nach der vorbestimmten Reaktionszeit das nachweisbare Signal gemessen. Das heißt, zur Erstellung eines Kalibrierungsgraphen wird jeweils eine gleiche Analyt-spezifische Substanz und ein jeweils gleicher Test-Analyt in unterschiedlichen Mengen verwendet. Vorzugsweise werden zur Erstellung eines Kalibrierungsgraphen 5 bis 50 unterschiedliche Mengen Test-Analyt, d.h. unterschiedliche Einzelreaktionen, stärker bevorzugt 10 bis 40 Einzelreaktionen und am stärksten bevorzugt 10 bis 25 Einzelreaktionen für entsprechend viele Zuordnungen von Test-Analytmenge zu Signal beschreibendem Parameter pro vorbestimmter Reaktionszeit durchgeführt.

Vor der Erstellung der Kalibrierungsgraphen können die experimentell ermittelten Werte auch einem kinetischen Auswertungsverfahren unterzogen werden, wobei zur Erstellung des Kalibrierungsgraphen dann durch das Auswertungsverfahren ermittelte Werte verwendet werden können

Der Test-Analyt und der quantitativ nachzuweisende Analyt sind vorzugsweise identisch.

Anhand der erstellten Kalibrierungsgraphen wird im erfindungsgemäßen Verfahren überprüft, ob für das gemessene Signal, welches aus der Reaktion der Analyt-spezifischen Substanz mit dem Analyten in der Probe resultiert, eine quantitative Bestimmung des Analyten mit einer gewünschte Genauigkeit erreicht wird. Für eine Überprüfung der Genauigkeit können beliebige im Fachbereich bekannte Auswertungsverfahren verwendet werden, die Signalhub oder Präzision berücksichtigen.

In einer bevorzugten Ausführungsform der vorliegenden Erfindung wird das nach einer vorbestimmten Reaktionszeit zwischen Analyt und Analyt-spezifischer Substanz gemessene Signal mit dem für die entsprechende vorbestimmte Reaktionszeit vorgegeben Kalibrierungsgraphen verglichen. Die gewünschte Genauigkeit für die zu bestimmende Analytmenge wird dabei erreicht, wenn der betrachtete Kalibrierungsgraph in Bezug auf die entsprechende Menge Test-Analyt die größte Steigung aller vorgegebenen Kalibrierungskurven aufweist.

Die wenigstens zwei vorgegebenen Reaktionszeiten zur Bestimmung der Kalibrierungsgraphen werden so gewählt, dass sowohl eine Detektion höherer Konzentrationen an Analyt in der Probe als auch eine nötige Testsensitivität erreicht wird. Zur Erreichung der nötigen Testsensitivität ist eine möglichst lange Reaktionszeit notwendig. Bei kürzeren Reaktionszeiten werden weniger Komplexe zwischen Analyt-spezifischer Substanz und Analyt, vorzugsweise Immunkomplexe gebildet. Es wird eine entsprechend niedrigere Signalintensität detektiert. Bei niedrigen Analyt-Konzentrationen werden hingegen zu wenige Komplexe, vorzugsweise Immunkomplexe gebildet und die Empfindlichkeit des Tests geht verloren. Bei hohen Konzentrationen stehen allerdings wesentlich mehr Komplexe zur Verfügung so dass auch bei kurzen Reaktionszeiten deutliche Signale bzw. hohe Signalintensitäten detektiert werden können. Durch die Kombination einer langen Reaktionszeit, welche die nötige Empfindlichkeit sicher stellt und einer kurzen Reaktionszeit, die der Detektion einer höheren Konzentration dient, wird der quantitative Messbereich der Reaktion zwischen Analyt und Analyt-spezifischer Substanz deutlich erhöht.

Bevorzugt wird deshalb als eine vorherbestimmte Reaktionszeit eine im Bezug auf die Reaktion von Analyt und Analyt-spezifischer Substanz lange Reaktionszeit gewählt, nach der sich die Reaktion zwischen Analyt und Analyt-spezifischer Substanz in einem Sättigungsbereich oder einem stationären Zustand befindet. Weitere vorbestimmte Reaktionszeiten werden bevorzugt entsprechend kürzer gewählt, so dass sich die Reaktion zwischen Analyt-spezifischer Substanz und Analyt zu diesen vorbestimmten kurzen Reaktionszeiten nicht in einem Sättigungsbereich oder einem stationären Zustand befindet. Vorzugsweise wird als wenigstens eine kurze Reaktionszeit eine Zeit gewählt, die in etwa der Hälfte der langen Reaktionszeit entspricht.

Die Analyt-spezifische Substanz wird auf einem Teststreifen oder Schnellteststreifen bereitgestellt.

Auf dem Teststreifen sind die zur Bestimmung des Analyten verwendeten Analyt-spezifischen Substanzen, bzw. Reagenzien in trockener - und nach Kontakt mit der Probe auflösbarer - Form in einer oder mehreren Zonen angeordnet, wobei die Erfassung des nachweisbaren Signals in einer Nachweiszone, vorzugsweise in einem separaten Bereich der Nachweiszone erfolgt. Im erfindungsgemäßen Verfahren können insbesondere alle kommerziell erhältlichen Teststreifen verwendet werden, welche zur quantitativen Bestimmung eines Analyten nach einem vorgegebenen festen Zeitwert geeignet sind.

Die Messbereichsgrenzen des eingesetzten Teststreifens können durch das erfindungsgemäße Verfahren um einen Faktor von 2 bis 5 und vorzugsweise mehr als 3 erweitert werden.

Nach einem weiteren Aspekt der vorliegenden Erfindung werden neben dem quantitativ zu bestimmenden Analyten weitere Analyten qualitativ und/oder quantitativ auf dem gleichen Träger bestimmt. Auf dem Träger liegen dann entsprechend mehrere jeweils Analyt-spezifische Substanzen vor. Hierbei kann es ferner zweckmäßig sein, an Analyt-spezifische Substanzen gekoppelte Nachweisreagenzien zu verwenden, die eine Bestimmung aller Analyten, quantitativ oder qualitativ durch ein einziges Testformat ermöglichen, beispielsweise einem Enzymtest, einen Elektrochemilumineszenztest, einem Fluoreszens- oder Absorptionstest, oder einen turbidimetrischen Test. Es können selbstverständlich aber auch verschiedene Nachweisreagenzien an die unterschiedlichen Analytspezischen Substanzen auf einem einzigen Teststreifen vorliegen.

In einer besonders bevorzugten Ausführungsform wird der Roche-CARDIAC-proBNP-Teststreifen verwendet.

So kann durch das erfindungsgemäße Verfahren der Messbereich des Roche-CARDIAC-proBNP-Teststreifens welcher in einem Bereich von 60-3000 pg/mL liegt, auf einen Bereich von 60-10000 pg/mL erweitert werden. Eine bevorzugte Ausführungsform der vorliegenden Erfindung betrifft deshalb die quantitative Bestimmung einer Analyt-Konzentration, wobei der Analyt wiederum vorzugsweise ausgewählt ist aus Troponin T, Myoglobin, D-Dimer und NT-proBNP. Im Fall von NT-proBNP erfolgt die Bestimmung beispielsweise im Bereich von 3000-10000 pg/mL bei einer kurzen Reaktionszeit zwischen Analyt-spezifischer Substanz und Analyt von 3-9 Minuten vorzugsweise 8 Minuten. Die Bestimmung von NT-proBNP im Konzentrationsbereich von 60-3000 pg/mL erfolgt vorzugsweise mit einer langen Reaktionszeit von 10-15 Minuten, bevorzugt 12 Minuten.

Die quantitative Bestimmung des nachweisbaren Signals, welches sich durch die Reaktion zwischen Analyt und Analyt-spezifischer Substanz ergibt, erfolgt vorzugsweise durch optische Methoden, insbesondere durch reflektionsphotometrischen oder fluorimetrischen Nachweis oder Elektrochemie-Lumineszenz. Weitere bevorzugte quantitative Bestimmungsverfahren umfassen Messungen zur Änderung von Dielektrizitätskonstanten, Leitfähigkeitsmessungen, Änderungen von Magnetfeldern, oder eine Änderung des Winkels der optischen Drehung von polarisiertem Licht.

In einer weiteren bevorzugten Ausführungsform der vorliegenden Erfindung wird das Verfahren automatisiert durchgeführt, beispielsweise in einem Analyseautomaten.

Ein weiterer Aspekt der vorliegenden Erfindung betrifft eine Vorrichtung zur Ausführung des erfindungsgemäßen Verfahrens. Diese Vorrichtung umfasst ein Speicherelement, auf welchem die für einen Analyten einmal erstellten Kalibrierungsgraphen gespeichert sind. Beispiel für ein Speicherelement umfassen alle geläufigen Datenträger wie ROM-Keys, Festplatten, CDs, Disketten, DVDs, USB-Sticks usw. Die gespeicherten Kalibrierungsgraphen können dann zur aufeinanderfolgenden quantitativen Bestimmung mehrerer Analyt-Proben vorgegeben werden.

Das erfindungsgemäße Verfahren kann insbesondere zur Identifizierung von Patienten mit akutem Koronarsyndrom eingesetzt werden, insbesondere zur Verbesserung der Früherkennung von akuten koronaren Ereignissen, beispielsweise zur Verbesserung der Früherkennung bei akutem Myokardinfarkt.

Weiterhin soll die vorliegende Erfindung durch das nachfolgende Beispiel erläutert werden.

### Abbildungen

Figur 1:
   Remissionskinetik von CARDIAC-proBNP nach 6 Min., 8 Min. und 12 Min.. Die Remission [%] ist gegen die Konzentration an proBNP [pg/mL] welche durch den Elecsys-proBNP-Referenztest bestimmt wurde, aufgetragen.
Figur 2:
   Methodenvergleich zwischen dem erfindungsgemäßen Verfahren unter Verwendung eines CARDIAC-proBNP-Teststreifens und dem Elecsys-proBNP-Test.

### Beispiele:

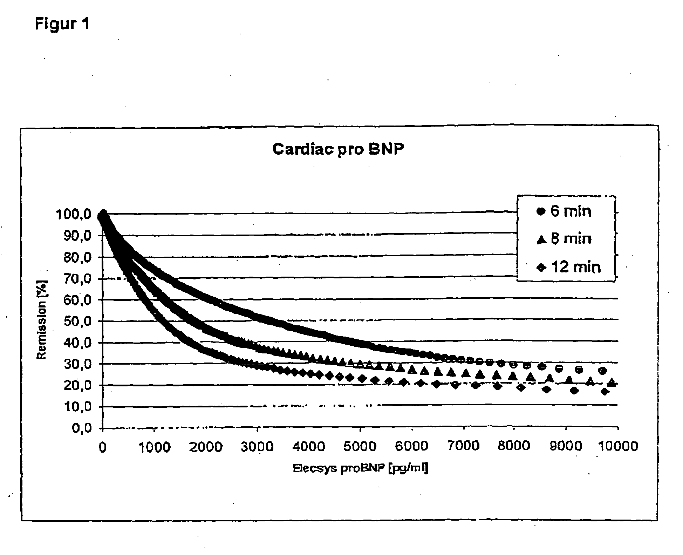

Unter Verwendung eines CARDIAC-proBNP-Teststreifens wurden Kalibrierungsgraphen nach 6, 8 und 12 Minuten Reaktionszeit erstellt. Die jeweiligen proBNP-Mengen wurden durch die Referenzmethode Elecsys-proBNP bestimmt. (Figur 1). Den Kalibrierungsgraphen ist zu entnehmen, dass ihre Steigung mit abnehmender Reaktionszeit abnimmt. Dies hat zur Folge, dass bei höheren Konzentrationen größer 3000 pg/mL der Signalhub und damit die Empfindlichkeit zunimmt.

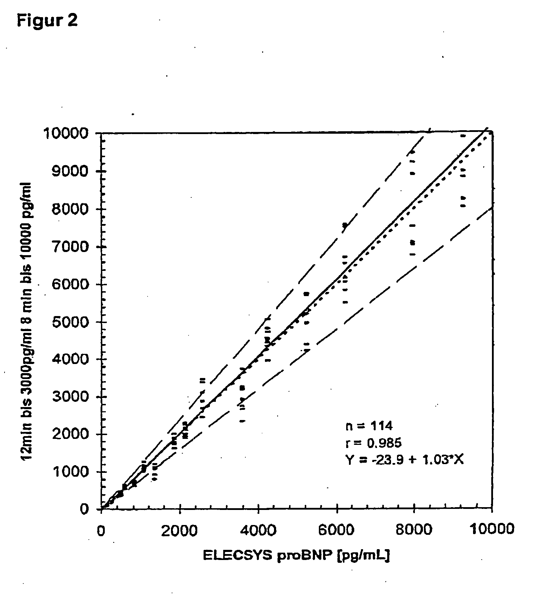

Durch Auswertung der Konzentrationsbereiche von 60-2800 pg/mL nach 12 Minuten und Konzentrationen größer 2800 pg/mL (das entspricht einem empirisch festgelegten oder definierten Remissionswert) nach 8 Minuten erhält man einen Methodenvergleich zwischen den erfindungsgemäß eingesetzten CARDIAC-proBNP-Test und dem Elecsys-proBNP-Test (Figur 2).

Beim Vergleich der Messungen zur Referenzmethode Elecsys-proBNP hat der mit den zwei unterschiedlichen Reaktionszeiten erfindungsgemäß ausgeführte CARCIAC-proBNP-Test einen quantitativen Messbereich von 60-ca. 10000 pg/mL. Im Vergleich zur herkömmlichen Auswertung nach 12 Minuten, welche eine obere Messbereichsgrenze von 3000 pg/mL aufweist, wurde die obere Messbereichsgrenze somit um mehr als das Dreifache erhöht.

## Patentansprüche

1. Verfahren zur quantitativen Bestimmung eines Analyten in einer Probe, umfassend
(a) Bereitstellen einer Analyt-spezifischen Substanz, die in der Lage ist, bei Kontakt mit dem Analyten eine Reaktion einzugehen, welche ein nachweisbares Signal erzeugt, wobei zum Bereitstellen der Analyt-spezifischen Substanz ein Teststreifen verwendet wird,
(b) Vorgeben von wenigstens zwei Kalibrierungsgraphen, die durch Reaktion der jeweils gleichen Analyt-spezifischen Substanz mit unterschiedlichen Mengen eines jeweils gleichen Test-Analyten für jeweils eine vorbestimmte Reaktionszeit erstellt worden sind,
(c) Inkontaktbringen der Analyt-spezifischen Substanz mit einer Probe, die den nachzuweisenden Analyten umfasst,
(d) Messen des Signals zu einer ersten vorbestimmten Reaktionszeit, für die ein erster Kalibrierungsgraph gemäß (b) vorgegeben ist,
(e) Überprüfen, ob das gemäß (d) gemessene Signal eine quantitative Bestimmung des Analyten mit einer gewünschten Genauigkeit ermöglicht, wobei die gewünschte Genauigkeit für die zu bestimmende Analytmenge erreicht ist, wenn der betrachtete Kalibierungsgraph in Bezug auf die entsprechende Menge Test-Analyt die größte Steigung aller vorgegebenen Kalibierungskurven aufweist,
(f)
(i) quantitatives Bestimmen des Analyten auf Basis des gemäß (d) gemessenen Signals, wenn die gewünschte Genauigkeit erreicht ist, wobei zum quantitativen Bestimmen des Analyten das gemäß (d) gemessene Signal mit dem für die erste vorbestimmte Reaktionszeit vorgegebenen ersten Kalibierungsgraphen verglichen wird,
oder
(ii) Messen des Signals zu einer zweiten vorbestimmten Reaktionszeit, für die ein zweiter Kalibrierungsgraph gemäß (b) vorgegeben ist,
(g) Überprüfen, ob das gemäß (f (ii)) gemessene Signal eine quantitative Bestimmung des Analyten mit einer gewünschten Genauigkeit ermöglicht, wobei die gewünschte Genauigkeit für die zu bestimmende Analytmenge erreicht ist, wenn der betrachtete Kalibierungsgraph in Bezug auf die entsprechende Menge Test-Analyt die größte Steigung aller vorgegebenen Kalibierungskurven aufweist, und
(h)
(i) quantitatives Bestimmen des Analyten auf Basis des gemäß f (ii) gemessenen Signals, wenn die gewünschte Genauigkeit erreicht ist, wobei zum quantitativen Bestimmen des Analyten das gemäß (f)(ii) gemessene Signal mit dem für die zweite vorbestimmte Reaktionszeit vorgegebenen zweiten Kalibierungsgraphen verglichen wird,
oder
(ii) Fortsetzen der Bestimmung bei zumindest einer weiteren vorbestimmten Reaktionszeit entsprechend (f) (ii), (g), (h)(i).

2. Verfahren zur quantitativen Bestimmung eines Analyten in einer Probe, umfassend
(a) Bereitstellen einer Analyt-spezifischen Substanz, die in der Lage ist, bei Kontakt mit dem Analyten eine Reaktion einzugehen, welche ein nachweisbares Signal erzeugt, wobei zum Bereitstellen der Analyt-spezifischen Substanz ein Teststreifen verwendet wird,
(b) Vorgeben von wenigstens zwei Kalibrierungsgraphen, die durch Reaktion der jeweils gleichen Analyt-spezifischen Substanz mit unterschiedlichen Mengen eines jeweils gleichen Test-Analyten für jeweils eine vorbestimmte Reaktionszeit erstellt worden sind,
(c) Inkontaktbringen der Analyt-spezifischen Substanz mit einer Probe, die den nachzuweisenden Analyten umfasst,
(d) Messen eines ersten Signals zu einer ersten vorbestimmten Reaktionszeit, für die ein erster Kalibrierungsgraph gemäß (b) vorgegeben ist,
(e) Messen eines zweiten Signals zu einer zweiten vorbestimmten Reaktionszeit, für die ein zweiter Kalibrierungsgraph gemäß (b) vorgegeben ist,
(f) gegebenenfalls Messen eines weiteren Signals,
(g) Überprüfen, welches der gemäß (d), (e) oder (f) gemessenen Signale eine ausreichende Genauigkeit bei der quantitativen Bestimmung des Analyten ermöglicht, wobei die gewünschte Genauigkeit für die zu bestimmende Analytmenge erreicht ist, wenn der betrachtete Kalibierungsgraph in Bezug auf die entsprechende Menge Test-Analyt die größte Steigung aller vorgegebenen Kalibierungskurven aufweist, und
(h) quantitatives Bestimmen des Analyten auf Basis des Signals, welches eine ausreichende Genauigkeit ermöglicht, wobei zum quantitativen Bestimmen des Analyten dieses Signal mit dem Kalibierungsgraphen verglichen wird, welcher für die entsprechende Reaktionszeit dieses Signals erstellt worden ist.

3. Verfahren nach Anspruch 1 oder Anspruch 2, **dadurch gekennzeichnet, dass** die Genauigkeit der quantitativen Bestimmung des Analyten mittels der Steigung der Kalibrierungsgraphen überprüft wird.

4. Verfahren nach Anspruch 1 oder 3, **dadurch gekennzeichnet, dass** der Test-Analyt und der quantitativ zu bestimmende Analyt identisch sind.

5. Verfahren nach einem der Ansprüche 1 bis 4, **dadurch gekennzeichnet, dass** zwei oder drei Kalibrierungsgraphen bestimmt werden.

6. Verfahren nach einem der Ansprüche 1 bis 5, **dadurch gekennzeichnet, dass** die Messbereichsgrenzen für den zu bestimmenden Analyten um einen Faktor 2 bis 5, vorzugsweise mehr als drei, erweitert werden.

7. Verfahren nach einem der Ansprüche 1 bis 6, **dadurch gekennzeichnet, dass** eine aus Körperflüssigkeit stammende Probe, vorzugsweise eine Blut-, Plasma-, Serum-, Speichel- oder Urinprobe verwendet wird.

8. Verfahren nach einem der Ansprüche 1 bis 7, **dadurch gekennzeichnet, dass** gleichzeitig mit dem quantitativ zu bestimmenden Analyten weitere Analyten qualitativ und/oder quantitativ bestimmt werden.

9. Verfahren nach einem der Ansprüche 1 bis 8, **dadurch gekennzeichnet, dass** der Analyt ausgewählt ist aus Nukleinsäuren, Lipiden, Kohlenhydraten Proteinen, und insbesondere aus DNA, RNA, Antikörpern, Antigenen, Stoffwechselprodukten, Hormonen Viren, Mikroorganismen, Zellen, kardiospezifischen Markern, neurohormonalen Markern ischämischen Markern und muskelspezifischen Markern.

10. Verfahren nach einem der Ansprüche 1 bis 9, **dadurch gekennzeichnet, dass** mindestens ein kardiospezifischer Marker ausgewählt aus Troponin T, Myoglobin, D-Dimer und NT-proBNP bestimmt wird.

11. Verfahren nach einem der Ansprüche 1 bis 10, **dadurch gekennzeichnet, dass** die Analyt-spezifische Substanz ausgewählt ist aus Antikörpern, mit dem Analyt bindefähigen Rezeptoren, Antigenen, Lectin, Nukleinsäuren und Nukleinsäureanalogen, wobei die Analyt-spezifische Substanz vorzugsweise mit einem Nachweisreagenz gekoppelt ist.

12. Verfahren nach einem der Ansprüche 1 bis 11, **dadurch gekennzeichnet, dass** die Reaktion zwischen Analyt und Analyt-spezifischer Substanz eine Immunreaktion ist.

13. Verfahren nach einem der Ansprüche 1 bis 12, **dadurch gekennzeichnet, dass** die quantitative Bestimmung durch optische Methoden, insbesondere durch reflexionsphotometrischen oder fluorimetrischen Nachweis oder Elektrochemilumineszenz erfolgt.

14. Verfahren nach einem der Ansprüche 1 bis 13, **dadurch gekennzeichnet, dass** das Verfahren automatisiert abläuft.

## Claims

1. A method for the quantitative determination of an analyte in a sample, said method comprising the following steps:
(a) providing an analyte-specific substance capable, on contact with the analyte, of undergoing a reaction which generates a detectable signal, a test strip being used for provision of the analyte-specific substance,
(b) provision of at least two calibration graphs, which have in each case been generated by reacting the same analyte-specific substance with different quantities of the same test analyte respectively, in each case for a predetermined reaction time,
(c) bringing the analyte-specific substance into contact with a sample containing the analyte to be detected,
(d) measuring the signal for a first predetermined reaction time, for which a first calibration graph is provided according to (b),
(e) checking whether the signal measured according to (d) permits a quantitative determination of the analyte with a desired accuracy, the desired accuracy in respect of the quantity of analyte to be determined being achieved if the calibration graph under consideration has the greatest slope of all the provided calibration curves in respect of the corresponding quantity of test analyte,
(f)
(i) quantitatively determining the analyte based on the signal measured according to (d), if the desired accuracy is achieved, in which case, for quantitative determination of the analyte, the signal measured according to (d) is compared with the first calibration graph provided for the first predetermined reaction time, or
(ii) measuring the signal for a second predetermined reaction time, for which a second calibration graph is provided according to (b),
(g) checking whether the signal measured according to (f (ii)) permits a quantitative determination of the analyte with a desired accuracy, the desired accuracy in respect of the quantity of analyte to be determined being achieved if the calibration graph under consideration has the greatest slope of all the provided calibration curves with regard to the corresponding quantity of test analyte, and
(h)
(i) quantitatively determining the analyte on the basis of the signal measured according to (f (ii)), if the desired accuracy is achieved, in which case, for quantitatively determining the analyte, the signal measured according to (f (ii)) is compared with the second calibration graph provided for the second predetermined reaction time,
or
(ii) continuing the determination with at least one more predetermined reaction time in accordance with (f) (ii), (g), (h) (i).

2. A method for the quantitative determination of an analyte in a sample, said method comprising the following steps:
(a) providing an analyte-specific substance capable, on contact with the analyte, of undergoing a reaction which generates a detectable signal, a test strip being used for provision of the analyte-specific substance,
(b) providing at least two calibration graphs, which have in each case been generated by reacting the same analyte-specific substance with different quantities of the same test analyte respectively, in each case for a predetermined reaction time,
(c) bringing the analyte-specific substance into contact with a sample containing the analyte to be detected,
(d) measuring a first signal for a first predetermined reaction time, for which a first calibration graph is provided according to (b),
(e) measuring a second signal for a second predetermined reaction time, for which a second calibration graph is provided according to (b),
(f) if necessary, measuring a further signal,
(g) checking which one of the signals measured according to (d), (e) or (f) permits adequate accuracy for quantitative determination of the analyte, the desired accuracy in respect of the quantity of analyte to be determined being achieved if the calibration graph under consideration has the greatest slope of all the provided calibration curves with regard to the corresponding quantity of test analyte, and
(h) quantitatively determining the analyte on the basis of the signal which permits sufficient accuracy, in which case, for quantitatively determining the analyte, this signal is compared with the calibration graph which has been generated for the corresponding reaction time of this signal.

3. A method according to Claim 1 or 2, **characterized in that** the accuracy of the quantitative determination of the analyte is checked using the slope of the calibration graphs.

4. A method according to Claim 1 or 3, **characterized in that** the test analyte and the analyte to be quantitively determined are identical.

5. A method according to one of Claims 1 to 4, **characterized in that** two or three calibration graphs are plotted.

6. A method according to one of Claims 1 to 5, **characterized in that** the limits of the measuring range for the analyte to be determined are extended by a factor of 2 to 5, and preferably more than 3.

7. A method according to one of Claims 1 to 6, **characterized in that** a sample derived from body fluid is used, and is preferably a blood, plasma, serum, saliva or urine sample.

8. A method according to one of Claims 1 to 7, **characterized in that**, at the same time as the analyte to be quantitatively determined, further analytes are qualitatively and/or quantitatively determined.

9. A method according to one of Claims 1 to 8, **characterized in that** the analyte is selected from nucleic acids, lipids, carbohydrates, proteins, and in particular from DNA, RNA, antibodies, antigens, metabolic products, hormones, viruses, microorganisms, cells, cardiospecific markers, neurohormonal markers, ischaemic markers and muscle-specific markers.

10. A method according to one of Claims 1 to 9, **characterized in that** at least one cardiospecific marker selected from troponin T, myoglobin, D-dimer and NT-proBNP is determined.

11. A method according to one of Claims 1 to 10, **characterized in that** the analyte-specific substance is selected from antibodies, receptors capable of binding with the analyte, antigens, lectin, nucleic acids and nucleic acid analogues, the analyte-specific substance preferably being coupled to a detection reagent.

12. A method according to one of Claims 1 to 11, **characterized in that** the reaction between analyte and analyte-specific substance is an immune reaction.

13. A method according to one of Claims 1 to 12, **characterized in that** the quantitative determination is achieved by optical methods, in particular by reflection-photometric or fluorimetric detection or by electrochemiluminescence.

14. A method according to one of Claims 1 to 13, **characterized in that** the method is automated.

## Revendications

1. Procédé pour la détermination quantitative d'un analyte dans un échantillon, comprenant les étapes de
(a) préparer une substance spécifique à l'analyte qui est capable d'entrer en réaction lors du contact avec l'analyte, qui produit un signal détectable, dans lequel une bandelette de test est utilisée pour préparer la substance spécifique à l'analyte,
(b) déterminer au moins deux courbes d'étalonnage, qui ont été préparées par réaction de chaque même substance spécifique de l'analyte respectivement avec des quantités différentes d'un même analyte de test, pour un temps de réaction prédéterminé,
(c) mettre en contact la substance spécifique à l'analyte avec un échantillon comprenant l'analyte à détecter,
(d) mesurer le signal à un premier temps de réaction prédéterminé, pour lequel une première courbe d'étalonnage est prédéterminée conformément à (b)
(e) vérifier que le signal mesuré conformément à (d) permet une détermination quantitative de l'analyte avec une précision souhaitée, dans lequel la précision de la quantité de l'analyte à déterminer est atteinte, quand la courbe d'étalonnage considérée possède la pente la plus importante de toutes les courbes d'étalonnage déterminées par rapport à la quantité correspondante de l'analyte test,
(f)
(i) déterminer quantitativement l'analyte sur la base du signal mesuré conformément à (d), quand la précision souhaitée est atteinte, dans lequel le signal mesuré conformément à (d) est comparé avec la première courbe d'étalonnage déterminée pour le premier temps de réaction prédéterminé, ou
(ii) mesurer le signal à un deuxième temps de réponse prédéterminé, pour lequel une seconde courbe d'étalonnage est déterminée conformément à (b),
(g) contrôler que le signal mesuré conformément à (f)(ii) permet une détermination quantitative de l'analyte avec une précision souhaitée, dans lequel la précision de la quantité de l'analyte à déterminer est atteinte, quand la courbe d'étalonnage considérée possède la pente la plus importante de toutes les courbes d'étalonnage déterminées par rapport à la quantité correspondante de l'analyte test, et
(h)
(i) déterminer quantitativement l'analyte sur la base du signal mesuré conformément au point (f)(ii), quand la précision souhaitée est atteinte, dans lequel le signal mesuré conformément à (d) est comparé avec la deuxième courbe d'étalonnage déterminée pour le deuxième temps de réaction prédéterminé, ou
(ii) poursuivre la détermination pour au moins un autre temps de réaction prédéterminé correspondant à (f) (ii), (g), (h) (i).

2. Procédé pour la détermination quantitative d'un analyte dans un échantillon, comprenant les étapes de
(a) préparer une substance spécifique à l'analyte qui est capable d'entrer en réaction lors du contact avec l'analyte, qui produit un signal détectable, dans lequel une bandelette de test est utilisée pour préparer la substance spécifique à l'analyte,
(b) déterminer au moins deux courbes d'étalonnage, qui ont été préparées par réaction de chaque même substance spécifique de l'analyte respectivement avec des quantités différentes d'un même analyte de test, pour un temps de réaction prédéterminé,
(c) mettre en contact la substance spécifique à l'analyte avec un échantillon comprenant l'analyte à détecter,
(d) mesurer un premier signal à un premier temps de réponse prédéterminé, pour lequel une première courbe d'étalonnage est déterminée conformément à (b),
(e) mesurer un deuxième signal à un deuxième temps de réponse prédéterminé, pour une seconde courbe d'étalonnage déterminée conformément à (b),
(f) facultativement, mesurer un autre signal,
(g) contrôler lequel des signaux mesurés conformément à (d), (e) ou (f) permet une précision suffisante dans la détermination quantitative de l'analyte, dans lequel la précision de la quantité de l'analyte à déterminer est atteinte, quand la courbe d'étalonnage considérée possède la pente la plus importante de toutes les courbes d'étalonnage déterminées par rapport à la quantité correspondante de l'analyte test, et
(h) déterminer quantitativement l'analyte en fonction du signal, qui permet d'atteindre une précision suffisante, dans lequel pour déterminer quantitativement l'analyte, ce signal est comparé avec la courbe d'étalonnage qui a été produite pour le temps de réaction correspondant à ce signal.
(i)

3. Procédé selon la revendication 1 ou la revendication 2, **caractérisé en ce que** la précision de la détermination quantitative de l'analyte est contrôlée au moyen de la pente de la courbe de calibration.

4. Procédé selon la revendication 1 ou 3, **caractérisé en ce que** l'analyte test et l'analyte quantitatif à déterminer sont identiques.

5. Procédé selon l'une quelconque des revendications 1 à 4, **caractérisé en ce que** deux ou trois courbes d'étalonnage sont déterminées.

6. Procédé selon l'une quelconque des revendications 1 à 5, **caractérisé en ce que** la limite du domaine de mesure pour l'analyte à déterminer est étendue d'un facteur 2 à 5, de préférence plus de trois.

7. Procédé selon l'une quelconque des revendications 1 à 6, **caractérisé en ce que** l'échantillon provenant d'un liquide corporel, de préférence un échantillon de sang, de plasma, de sérum, de salive ou d'urine est utilisé.

8. Procédé selon l'une quelconque des revendications 1 à 7, **caractérisé en ce que**, en même temps que l'analyte déterminer de façon quantitative, d'autres analytes sont déterminés de façon quantitative et/ou qualitative.

9. Procédé selon l'une quelconque des revendications 1 à 8, **caractérisé en ce que** l'analyte est choisi parmi les acides nucléiques, les lipides, les protéines glucidiques, et en particulier les ADN, ARN, anticorps, antigènes, produits de métabolisme, hormones, virus, microorganismes, cellules, marqueurs spécifiques du coeur, marqueurs neurohormonaux, marqueurs ischémiques et marqueurs spécifiques des muscles.

10. Procédé selon l'une quelconque des revendications 1 à 9, **caractérisé en ce que** au moins un marqueur spécifique du coeur, choisi parmi la troponine T, la myoglobine, le dimère D et NT-proBNP, est déterminé.

11. Procédé selon l'une quelconque des revendications 1 à 10, **caractérisé en ce que** la substance spécifique à l'analyte est choisie parmi les anticorps, les récepteurs capables de se lier à l'analyte, les antigènes, la lectine, les acides nucléiques et les analogues d'acides nucléiques dans lequel la substance spécifique à l'analyte est couplée de préférence avec un réactif de détection.

12. Procédé selon l'une quelconque des revendications 1 à 11, **caractérisé en ce que** la réaction entre l'analyte et la substance spécifique à l'analyte est une réaction immunitaire.

13. Procédé selon l'une quelconque des revendications 1 à 12, **caractérisé en ce que** la détermination quantitative est réalisée par des méthodes optiques, en particulier par détection de réflectométrie ou de fluorométrie ou par électrochimioluminescence.

14. Procédé selon l'une quelconque des revendications 1 à 13, **caractérisé en ce que** le procédé se déroule de façon automatisé.
